# EUROPEAN PATENT SPECIFICATION

(11) **EP 2 704 662 B1**
(45) Date of publication and mention of the grant of the patent: **12.10.2016**
(21) Application number: 12779456.8
(22) Date of filing: 04.05.2012
(51) Int. Cl.: A61C 5/00, A61K 49/04

(54) **USE OF RADIOGRAPHIC CONTRAST AGENTS FOR DETECTING DENTAL CARIES**
VERWENDUNG VON RÖNTGENKONTRASTMITTELN ZUR ERKENNUNG VON KARIES
UTILISATION D'AGENTS DE CONTRASTE RADIOGRAPHIQUE POUR DÉTECTER DES CARIES DENTAIRES

(30) Priority: 05.05.2011 US 201161482825 P; 27.09.2011 US 201161539744 P
(43) Date of publication of application: 12.03.2014
(73) Proprietor: Creighton University, Omaha, NE 68178 (US)
(72) Inventor: BENN, Douglas K., Omaha, Nebraska 68178 (US)
(74) Representative: Hamer, Christopher K.
(86) International application number: PCT/US2012/036444
(87) International publication number: WO 2012/151464

(56) References cited:
- WO-A1-98/27889
- US-A- 4 302 376
- US-A- 4 652 593
- US-A- 6 054 400
- US-A1- 2008 255 266
- US-A1- 2009 317 772
- VALIZADEH SOLMAZ ET AL: "Accuracy of digital subtraction radiography in combination with a contrast media in assessment of proximal caries depth", JOURNAL OF DENTAL RESEARCH, DENTAL CLINICS, DENTAL PROSPECTS, TABRIZ UNIVERSITY OF MEDICAL SCIENCES * FACULTY OF DENTISTRY, IR, vol. 2, no. 3, 1 January 2008 (2008-01-01) , pages 77-81, XP008172292, ISSN: 2008-210X, DOI: 10.5681/JODDD.2008.016PUBMED:23277849
- A. C Shearer ET AL: "The use of a radiopaque contrast medium in endodontic radiography", International Endodontic Journal, 1 January 1996 (1996-01-01), pages 95-98, XP055142465, Retrieved from the Internet: URL:http://onlinelibrary.wiley.com/store/1 0.1111/j.1365-2591.1996.tb01168.x/asset/j. 1365-2591.1996.tb01168.x.pdf?v=1&t=i0gt5ly 0&s=fd37cc6e2deb6984f87964e86d014a22ef4afb 43 [retrieved on 2014-09-24]
- DATABASE MEDLINE [Online] US NATIONAL LIBRARY OF MEDICINE (NLM), BETHESDA, MD, US; March 1998 (1998-03), YONG W ET AL: "[The radiopaque evaluation of NaI radiopaque resinifying agent in vitro].", XP002730175, Database accession no. NLM11774685 & YONG W ET AL: "[The radiopaque evaluation of NaI radiopaque resinifying agent in vitro].", ZHONGHUA KOU QIANG YI XUE ZA ZHI = ZHONGHUA KOUQIANG YIXUE ZAZHI = CHINESE JOURNAL OF STOMATOLOGY MAR 1998, vol. 33, no. 2, March 1998 (1998-03), pages 106-108, ISSN: 1002-0098
- Anonymous: "NCT01523509 on 2012_01_31 Use of radiographic contrast to detect dental caries", ClinicalTrials.gov Archive, 31 January 2012 (2012-01-31), XP055141650, Retrieved from the Internet: URL:http://clinicaltrials.gov/archive/NCT0 1523509/2012_01_31 [retrieved on 2014-09-19]
- Wilkinson Taylor G, Rocha-Sanchez S. et al.: "Sodium iodide radiographic contrast to distinguish between non- and cavitated carious lesions", Creighton University Medical Center , 28 March 2012 (2012-03-28), XP002730176, Retrieved from the Internet: URL:https://dspace.creighton.edu/xmlui/han dle/10504/27964 [retrieved on 2014-09-19]
- Wilkinson T. G.: "The Use of NaI As A Novel Radiographic Contrast Material For DifferentiatingBetween Cavitated and Non-cavitated Interproximal Carious Lesions Thesis", Creighton University , 29 March 2012 (2012-03-29), XP002730177, Retrieved from the Internet: URL:https://dspace.creighton.edu/xmlui/bit stream/handle/10504/28619/Wilkinson_Taylor _Thesis.pdf?sequence=1 [retrieved on 2014-09-25]
- Anonymous: "3.2 Stages in Caries Lesion Severity and Activity Cavitated Caries Lesions", School-Based Dental Sealant Programs, 1 January 2009 (2009-01-01), XP055215824, Retrieved from the Internet: URL:http://ohiodentalclinics.com/curricula /sealant/mod3_2_2.html [retrieved on 2015-09-24]
- Anonymous: "3.2 Stages in Caries Lesion Severity and Activity Non-Cavitated Caries Lesions", School-Based Dental Sealant Programs, 1 January 2009 (2009-01-01), pages 1-2, XP055215825, Retrieved from the Internet: URL:http://ohiodentalclinics.com/curricula /sealant/mod3_2_1.html [retrieved on 2015-09-24]

## Description

### FIELD OF THE INVENTION

This invention relates to a topical intra-oral solution comprising a iodide contrast agent for use in the *in vivo* diagnosis of cavitated dental caries, as defined in the claims.

### BACKGROUND OF THE INVENTION

Dental caries, caused by a variety of sources, is a common disease that can be easily treated if detected early. If undetected and untreated, caries may progress through the outer enamel layer of a tooth into the dentin and pulp leading to apical tissue inflammation so far as to require extraction of the tooth. The standard methods for detecting caries in teeth are by visual inspection or by the use of dental x-rays.

Both methods of visual inspection and x-ray imaging are incapable of differentiating between cavitated and non-cavitated dental carious lesions between the posterior teeth, especially in and around the areas where the teeth contact one another (the interproximal region). These methods are also incapable of accurately differentiating between dental caries and the shapes of the pits and fissures characteristic of the biting and chewing surfaces of the teeth (the occlusal surfaces). The difference between cavitated and non-cavitated lesions is clinically significant, as non-cavitated lesions may not progress, and may not require intervention, such as a composite or metallic filling. Thus, there is a need in the art for a method of obtaining enhanced images of dental caries, especially between teeth, where visual inspection is not possible.

Valizadeh et al. disclose in J. Dental Research, Deantal Clinics, Dental Prospects, 2 (3), 77-81, 2008, the diagnosis of proximal dental caries using digital subtraction radiography in combination with barium sulfates.

US-A-2008/0255266 discloses a dental paste composition having X-ray imaging properties.

WO-A-9827889 discloses a radiopaque iodine solution for visualising root canal pathologies.

### SUMMARY OF THE INVENTION

The present invention provides a topical intra-oral solution comprising a contrasting agent for use in the *in vivo* diagnosis of cavitated dental caries, wherein the contrasting agent is a iodide compound, and wherein the topical intra-oral solution is administered to one or more teeth of a subject and a radiographic image of the one or more teeth is produced as part of the diagnosis. In some embodiments, the contrast agent may be selected from the group consisting of sodium iodide, potassium iodide, magnesium iodide, calcium iodide, diatrizoate meglumine, diatrizoate sodium, and combinations thereof. In one embodiment, the iodide compound is selected from the group consisting of sodium iodide and potassium iodide. Additionally, the pharmaceutically acceptable carrier may generally include distilled water, deionized water, purified water, and combinations thereof. The contrast may also be administered as a substantially pure powder.

The use includes the administration of a composition having an iodide compound concentration ranging from about 0.5 molar to about 9 molar, from about 2 molar to about 8 molar, from about 4 molar to about 7 molar, and from about 5.5 molar to about 6.5 molar. In one embodiment, the iodide compound is present in the topical intra-oral composition at a concentration of about 6 molar.

The use may include administering the topical intra-oral composition to one or more teeth by means of dropping, immersing, inserting, rinsing, spraying, air-blasting, brushing, swabbing, or combinations thereof. The topical intra-oral composition may further include additional components such as a binder, a flavoring agent, and a coloring agent.

In another aspect, the current disclosure provides a method of imaging dental caries, diagnosing or monitoring periodontal disease, and evaluating the 3D shape of dental root canals prior to root canal therapy in a human comprising the steps of: (a) administering a topical intra-oral composition comprising sodium iodide and distilled water to one or more teeth of the subject; and (b) producing a radiographic image of the one or more teeth, wherein the sodium iodide comprises a concentration ranging from about 0.5 molar to about 9 molar. Additionally, the sodium iodide may be present in the topical intra-oral composition at concentrations ranging from about 2 molar to about 8 molar, from about 4 molar to about 7 molar, from about 5.5 molar to about 6.5 molar, and about 6 molar.

In yet another aspect, the current disclosure provides a method of imaging dental caries, diagnosing or monitoring periodontal disease, and evaluating the 3D shape of dental root canals prior to root canal therapy in a human comprising the steps of administering a topical intra-oral composition of barium sulfate to one or more teeth of the subject, and producing a radiographic image of the one or more teeth, wherein the barium sulfate comprises a concentration ranging from about 80% w/w to about 100% w/w. Additionally, the barium sulfate may be present in the topical intra-oral composition at a concentration of about 96% w/w.

In still another aspect, the current disclosure provides a method of imaging dental caries, diagnosing or monitoring periodontal disease, and evaluating the 3D shape of dental root canals prior to root canal therapy in a human comprising the steps of administering a topical intra-oral composition comprising a combination of diatrizoate meglumine and diatrizoate sodium to one or more teeth of the subject, and producing a radiographic image of the one or more teeth.

In still another aspect, the current disclosure provides a topical intra-oral composition for enhancing radiographic contrast images, comprising: (a) an iodide compound; and (b) a pharmaceutically acceptable carrier. Generally, the iodide compound may include sodium iodide, potassium iodide, magnesium iodide, calcium iodide, diatrizoate meglumine, diatrizoate sodium, and combinations thereof. In one embodiment, the iodide compound is selected from the group consisting of sodium iodide and potassium iodide. Additionally, the pharmaceutically acceptable carrier may include distilled water, deionized water, purified water, and combinations thereof.

The iodide compound may be present in the topical intra-oral composition at concentrations ranging from about 0.5 molar to about 9 molar, from about 2 molar to about 8 molar, from about 4 molar to about 7 molar, and from about 5.5 molar to about 6.5 molar. In one embodiment, the iodide compound is present in the topical intra-oral composition at a concentration of about 6 molar.

Additionally, the topical intra-oral composition may be administered to one or more teeth by means of dropping, immersing, inserting, rinsing, spraying, air-blasting, brushing, swabbing, or combinations thereof. Further, in one aspect of the disclosure, the topical intra-oral composition may include a dosage form comprising a solution, a suspension, a dispersion, an emulsion, a solid, a paste, a foam, and a gel. The topical intra-oral composition may further include additional components such as a binder, a flavoring agent, and a coloring agent.

In a further aspect, the current disclosure provides a topical intra-oral composition comprising sodium iodide and distilled water, wherein the sodium iodide comprises a concentration ranging from about 0.5 molar to about 9 molar. Additionally, the sodium iodide may be present in the topical intra-oral composition at concentrations ranging from about 2 molar to about 8 molar, from about 4 molar to about 7 molar, from about 5.5 molar to about 6.5 molar, and about 6 molar.

In yet another aspect, the current disclosure provides a kit for enhancing radiographic images comprising: (a) a topical intra-oral composition including an iodide compound and a pharmaceutically acceptable carrier; and at least one application accessory selected from the group consisting of a syringe, a container, a sprayer, a brush, a swab, a tray, and combinations thereof.

Other aspects and features of the invention are detailed below. Subject-matter which is not encompassed by the scope of the claims does not form part of the present claimed invention.

### BRIEF DESCRIPTION OF DRAWINGS

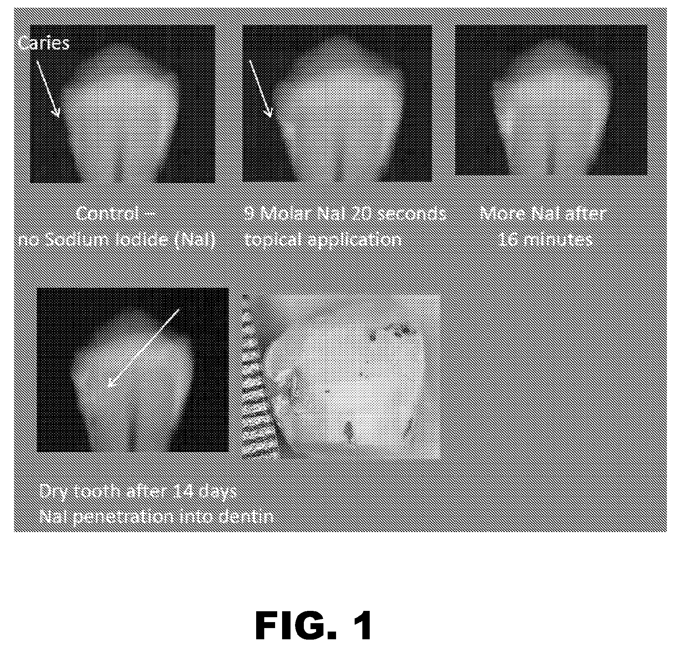

**FIG. 1** illustrates the absorption and subsequent imaging of a 9 M sodium iodide composition compared to a tooth imaged without the application of sodium iodide. Specifically, **FIG. 1** illustrates that a tooth administered a topical 9 M sodium iodide solution provided an enhanced radiographic image of dental caries after 20 seconds and 16 minutes that were not present in the image of the same tooth before the application of the solution.

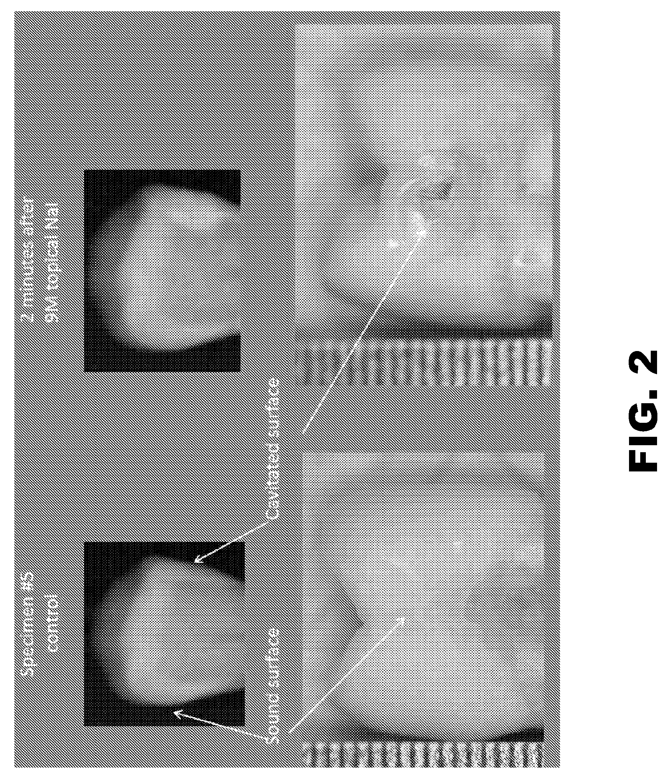

**FIG. 2** illustrates a different tooth and the absorption and subsequent imaging of a 9 M sodium iodide composition before and after the application of sodium iodide. Specifically, **FIG. 2** illustrates that a tooth administered a topical 9 M sodium iodide solution provided an enhanced radiographic image of dental caries after 2 minutes that were not present in the image of the same tooth before treatment with the iodide solution. **FIG. 2** also illustrates that the topical 9 M sodium iodide solution does not affect the imaging of sound tooth surfaces, indicating that only the images of cavitated surfaces are enhanced by administration of the sodium iodide solution.

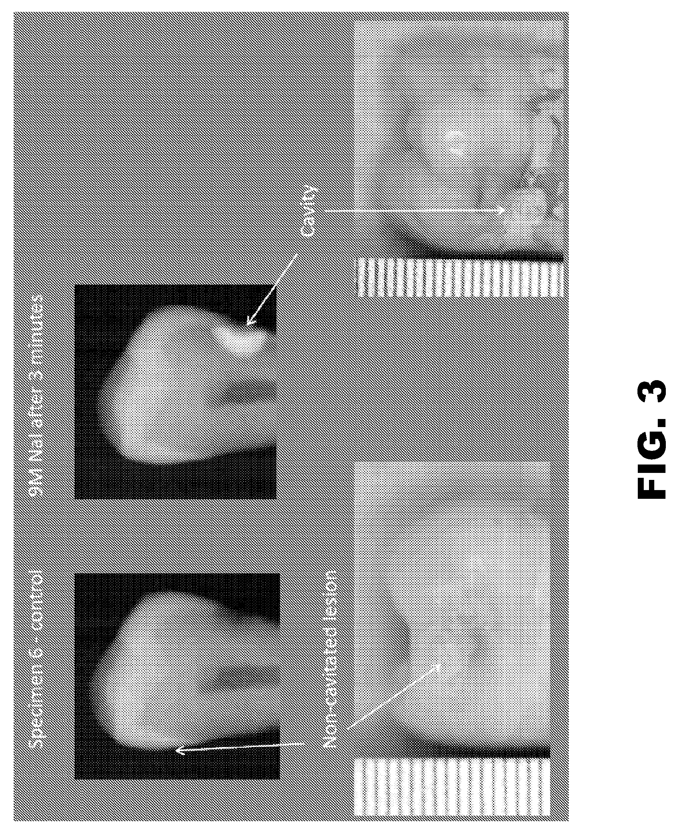

**FIG. 3** illustrates the absorption and subsequent imaging of a 9 M sodium iodide composition compared to the same tooth imaged without the application of sodium iodide. Specifically, **FIG. 3** illustrates that a tooth administered a topical 9 M sodium iodide solution provided an enhanced radiographic image of a cavitated lesion (cavity) after 3 minutes that were not present in the image of the same tooth previously before treatment with the iodide solution. **FIG. 3** also illustrates that the topical 9 M sodium iodide solution does not affect the imaging of non-cavitated lesions that may not need therapeutic intervention, indicating that only the images of cavitated surfaces are enhanced by administration of the sodium iodide solution.

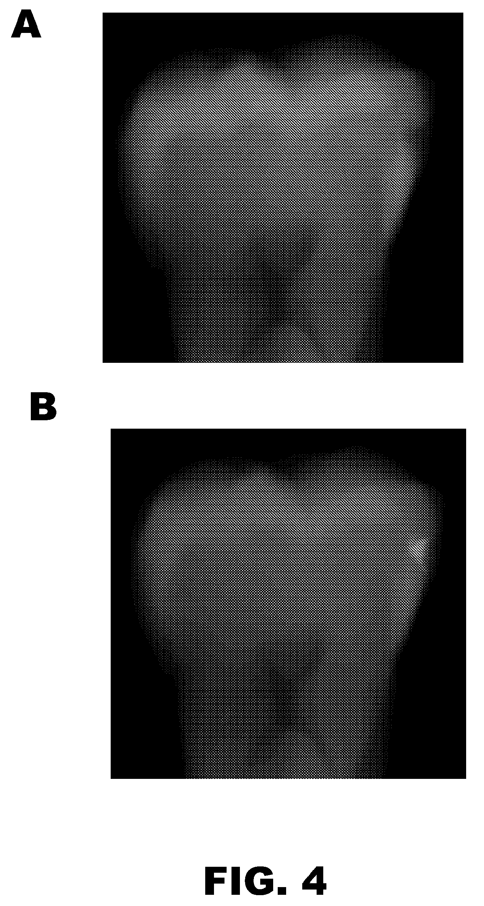

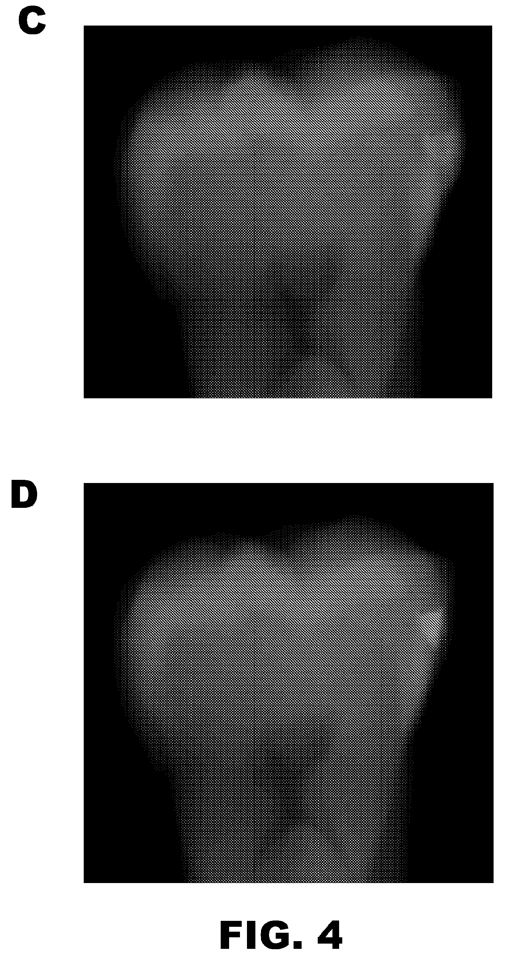

**FIG. 4** illustrates the absorption and subsequent imaging of a 9 M sodium iodide composition **(B),** a 76% solution of iopamidol **(C),** and a solution of barium sulfate (1 gram in 0.5 ml distilled water) (reference) (D), compared to the same tooth imaged without the application of imaging solutions **(A).**

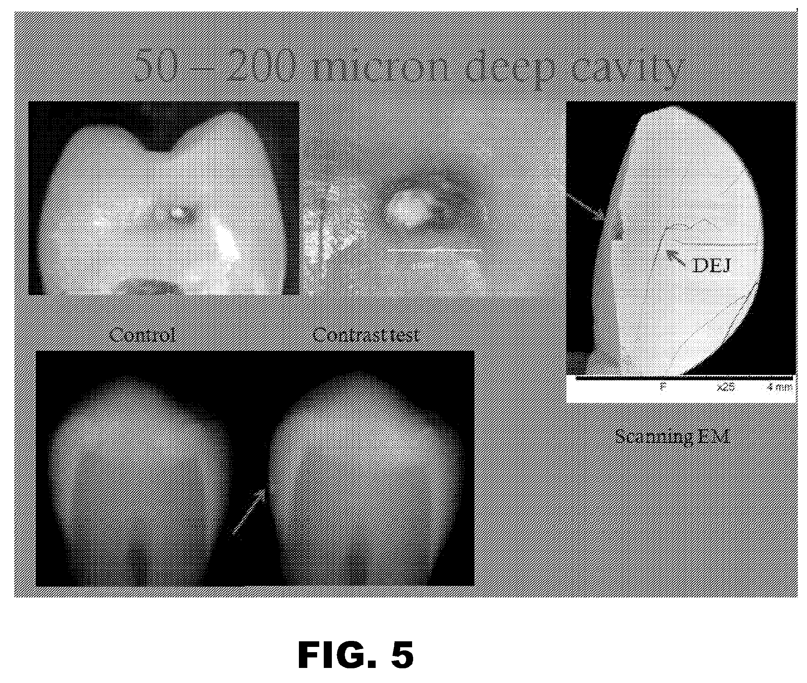

**FIG. 5** depicts an image showing a shallow cavity 50 - 200 microns deep. The radiograph was taken 15 seconds after barium sulfate (96% w/w) (reference) was applied. The cavity is marked with an arrow.

### DETAILED DESCRIPTION OF THE INVENTION

The present invention provides an efficient means for imaging dental caries that provides enhanced images capable of differentiating between cavitated dental carious lesions that may require therapeutic intervention and non-cavitated lesions that may not decay further and may not require therapeutic intervention. The topical intra-oral solutions used in the current invention are especially effective for imaging dental caries that are present in the spaces between teeth that are not accessible for visual examination and that generally cannot be adequately imaged by traditional methods, and for imaging dental caries that are present on or beneath the biting and chewing surfaces of the teeth and that are characterized by a complex anatomy of pits and fissures that generally makes it challenging to identify dental caries and distinguish that decay from staining and the shapes of the pits and fissures of the teeth.

The topical intra-oral solutions of the current invention are generally useful in distinguishing cavitated from non-cavitated dental caries, such that the practitioner may better monitor the subject's oral health and make informed decisions regarding caries treatment options. The radiographic image may be produced prior to, or after the determination that one or more suspected carious lesions are present. In one embodiment, the radiographic images of the current invention are utilized as a diagnostic tool to determine the presence or absence of cavitated decay in the one or more teeth of the subject. In another embodiment, the radiographic images of the current invention are generally used as a monitoring tool to monitor the progression or regression of cavitated lesions and to monitor non-cavitated lesions to determine if the lesions have progressed to cavitation.

The present disclosure also provides an efficient method for diagnosing or monitoring periodontal disease and an efficient method for evaluation of the 3D shape of dental root canals prior to root canal therapy.

Specifically, the present invention disclosure a method of imaging dental caries, diagnosing or monitoring periodontal disease, and evaluating the 3D shape of dental root canals prior to root canal therapy in a subject comprising the steps of: (a) administering a topical intra-oral composition comprising a contrast agent to one or more teeth of the subject, and (b) producing a radiographic image of the one or more teeth.

### I. Topical Intra-Oral Composition

As used herein, the term "topical intra-oral" is used to describe a route of administration whereby the composition is generally applied directly to the tooth or teeth sites intended to be imaged. The topical intra-oral compositions of the current invention are generally not swallowed or consumed by the subject, and the topical administration generally does not result in significant systemic absorption of the composition. In some embodiments, it may be possible that the composition is accidentally ingested by the subject; however, the composition is generally administered in small amounts that do not result in adverse effects.

A topical intra-oral composition comprises a contrast agent. The contrast agent of the invention is an iodide compound. The iodide compounds incorporated into the compositions of the current invention may be ionic or non-ionic compounds. In some embodiments, the iodide compounds may be an alkali metal salt of iodine. The ionic iodide compounds may include sodium iodide, potassium iodide, magnesium iodide, diatrizoate, metrizoate, ioxaglate and calcium iodide. In one embodiment, the iodide salt is selected from the group consisting of sodium iodide and potassium iodide.

In other embodiments, the iodide compounds of the invention may be non-ionic iodine compounds. Suitable examples may include iopamidol, iotrolan, iohexol, ioxilan, iopromide, iothalamate meglumine, iodixanol, diatrizoate meglumine, diatrizoate sodium, or combinations thereof. In one embodiment, the non-ionic iodine compound is iopamidol. In another embodiment, the non-ionic iodine compound is a combination of diatrizoate meglumine and diatrizoate sodium.

In some aspects of the disclosure, the topical intra-oral contrast composition may comprise contrast agents other than iodine. Non-limiting examples of contrast agents may include barium salts such as barium sulfate, calcium salts such as calcium hydroxide, calcium sulfate, calcium carbonate, calcium phosphate or calcium chloride, zinc salts such as zinc carbonate or zinc oxide, gold, diatrizoate meglumine, and diatrizoate sodium solution. In some aspects, the topical intra-oral contrast composition may comprise calcium hydroxide as a contrast agent. In other aspects, the topical intra-oral contrast composition may comprise zinc oxide as a contrast agent. In yet other aspects, the topical intra-oral contrast composition may comprise gold as a contrast agent. In some aspects, the topical intra-oral contrast composition of the invention may comprise barium sulfate as a contrast agent.

A topical intra-oral composition may include any concentration of the contrast agent necessary to adequately provide enhanced imaging, taking into consideration the solubility of the various composition constituents. Generally, when the contrast agent is an iodide compound, the concentration of the iodide compound may range from about 0.1 molar (M) to about 10 molar. In one embodiment, the composition has an iodide compound concentration ranging from about 0.5 molar to about 9 molar. In another embodiment, the composition has an iodide compound concentration ranging from about 2 molar to about 8 molar. In still another embodiment, the composition has an iodide compound concentration ranging from about 4 molar to about 7 molar. In an additional embodiment, the composition has an iodide compound concentration ranging from about 5.5 molar to about 6.5 molar. In a further embodiment, the iodide compound is present in the topical intra-oral composition at a concentration of about 6 molar.

When the contrast agent is a combination of diatrizoate meglumine and diatrizoate sodium, the concentration of iodine in the topical intra-oral composition may be about 100 mg/ml to about 600 mg/ml. In some embodiments, the concentration of iodine in the topical intra-oral composition may be about 100, 200, 300, 400, 500 or 600 mg/ml. In other embodiments, the concentration of iodine in the topical intra-oral composition may be about 310, 320, 330, 340, 350, 360, 370, 380, 390 or about 400 mg/ml. In an exemplary embodiment, the concentration of iodine in the topical intra-oral composition may be about 367 mg/ml. Examples of formulations comprising a combination of diatrizoate meglumine and diatrizoate sodium may include Gastrografin® provided by Bracco Diagnostics Inc., and MD-Gastroview® provided by Covidien.

In an aspect of the disclosure, when the contrast agent is a barium compound, such as barium sulfate, the concentration of the barium compound in the topical intra-oral composition may be about 40% w/w to about 65% w/w. In some aspects, the barium compound in the topical intra-oral composition may be about 40, 41, 42, 43, 44, 45, 46, 47, 48, 49, 50, 51, 52, 53, 54, 55, 56, 57, 58, 59, 60, 61, 62, 63, 64 or 65% w/w. In other aspects, the barium compound in the topical intra-oral composition may be about 65% w/v to about 120% w/v. For instance, the barium compound in the topical intra-oral composition may be about 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, or 120% w/v. In an exemplary aspect, the barium compound in the topical intra-oral composition is present at a concentration of about 96%. In an exemplary embodiment, the barium compound is suspended in water. In another exemplary aspect, the barium compound in the topical intra-oral composition may be administered as a substantially pure powder. For example, a substantially pure barium compound powder preferably comprises at least about 98%, more preferably at least about 99% by weight of barium compound of the total material in a given sample. In some aspects, the barium compound in the substantially pure powder may be about 98.1, 98.2, 98.3, 98.4, 98.5, 98.6, 98.7, 98.8, 98.9, 99, 99.1, 99.2, 99.3, 99.4, 99.5, 99.6, 99.7, 99.8, 99.9, or about 100% by weight of barium compound of the total material in a given sample. In other aspects, the barium compound in the substantially pure powder may be about 99, 99.1, 99.2, 99.3, 99.4, 99.5, 99.6, 99.7, 99.8, 99.9, 99.99, 99.999 or about 100% by weight of barium compound of the total material in a given sample.

The *in vivo* diagnosis of the current invention may further comprise cleaning the one or more teeth. Suitable methods of cleaning one or more teeth will be able to remove debris and other obstructions from pits and fissures of the one or more teeth so as to allow the topical intra-oral composition to pass beyond those obstructions and fill any cavities that exist. Methods of cleaning teeth are known in the art, and may include the use of a tooth-cleaning agent. Non limiting examples of tooth-cleaning agents may include barium sulfate, sodium bicarbonate, aluminum oxide, and particulate bioactive glass. In one embodiment, the teeth are cleaned with sodium bicarbonate. In another embodiment, the teeth are cleaned with aluminum oxide. In yet another embodiment, the teeth are cleaned with barium sulfate. In an exemplary embodiment, the teeth are cleaned with a tooth-cleaning particulate bioactive glass. The term "bioactive glass" as used herein describes a composition comprising silicon oxide (SiO₂) that may be used in conjunction with pressurized air to clean debris from teeth. Bioactive glass may be formulated as particles with an average particle size of less than 50 µm. Methods of cleaning teeth using a tooth-cleaning particulate bioactive glass are known to those skilled in the art and may be found in, for example, US6365132, US6190643, US6244871, US6086374 and WO1996010985.

In some embodiments, the one or more teeth are cleaned in conjunction with administration of the topical intra-oral composition comprising a contrast agent. The term "in conjunction with" as used herein indicates that the one or more teeth may be cleaned before, simultaneously with, or after administration of the composition comprising a contrast agent. In some embodiments, the one or more teeth are cleaned before administration of the topical intra-oral composition comprising a contrast agent. In other embodiments, the one or more teeth are cleaned after administration of the topical intra-oral composition comprising a contrast agent. In yet other embodiments, the contrast agent is administered simultaneously with the tooth-cleaning agent in a mixture comprising the contrast agent and the tooth-cleaning agent. In exemplary embodiments, the cleaning agent is bioactive glass, and the contrast agent is administered simultaneously with the tooth-cleaning particulate bioactive glass in a mixture comprising the contrast agent and the particulate bioactive glass. In some alternatives of the embodiments, the contrast agent is formulated with the tooth-cleaning particulate bioactive glass. Methods of formulating particulate bioactive glass are known to those skilled in the art and may be found in, for example, US6365132, US6190643, US6244871, US6086374 and WO1996010985.

A pharmaceutically acceptable carrier of the topical intra-oral composition may generally be defined as any carrier capable of delivering the contrast agent to the surface of the diagnostic area requiring imaging. Typical carriers may include solvent or solvent-like solutions such as water, organic solvents including alcohols (e.g. methyl alcohol, ethyl alcohol, n- and iso-propyl) and ketones (e.g acetone), and combinations thereof. Suitable examples of water carriers include deionized water, distilled water, and purified water.

Suitable organic solvents include, but are not limited to, alkane and substituted alkane solvents (including cycloalkanes) such as methanol, ethanol, isopropanol, n-propanol, isobutanol, n-butanol, s-butanol, t-butanol, formic acid, acetic acid, aromatic hydrocarbons, esters, ethers, ketones, combinations thereof, and the like. Specific organic solvents that may be employed, include, for example, acetonitrile, benzene, butyl acetate, t-butyl methylether, t-butyl methyl ketone, chlorobenzene, chloroform, chloromethane, cyclohexane, dichloromethane, dichloroethane, diethyl ether, ethyl acetate, diethylene glycol, fluorobenzene, heptane, hexane, isobutylmethylketone, isopropyl acetate, methylethylketone, methyltetrahydrofuran, pentyl acetate, n propyl acetate, toluene, acetonitrile, diethoxymethane, N,N-dimethylformamide (DMF), dimethyl sulfoxide (DMSO), N,N-dimethylpropionamide, 1,3-dimethyl-3,4,5,6-tetrahydro-2(1H)-pyrimidinone (DMPU), 1,3-dimethyl-2-imidazolidinone (DMI), 1,2-dimethoxyethane (DME), dimethoxymethane, bis(2-methoxyethyl)ether, N,N-dimethylacetamide (DMAC), 1,4-dioxane, N-methyl-2-pyrrolidinone (NMP), ethyl acetate, ethyl formate, ethyl methyl ketone, formamide, hexachloroacetone, hexamethylphosphoramide, methyl acetate, N-methylacetamide, N-methylformamide, methylene chloride, nitrobenzene, nitromethane, propionitrile, sulfolane, tetramethylurea, tetrahydrofuran (THF), 2-methyl tetrahydrofuran, trichloromethane, and combinations thereof.

In addition, as will be appreciated by a skilled artisan, the topical intra-oral compositions of the current invention may include a variety of suitable inert, physiologically acceptable excipients and diluents. Suitable inert excipients may include, but are not limited to surfactants, penetration enhancers, thickeners, buffers, propellants, binders, fillers, lubricants, diluents, flavor-modifying agents, sweeteners, coloring agents, and taste masking agents. As will be appreciated by a skilled artisan, the topical formulation may include a combination of any of the forgoing additional agents in varying amounts.

In one embodiment, the topical intra-oral composition may optionally include one or more surface-active agents (also called emulsifying agents). Emulsifiers and surfactants are generally used in preparing those embodiments of the present invention directed to compositions that are formulated as emulsions. Either water in oil or oil in water emulsions may be formulated. Examples of suitable surfactants and emulsifying agents include: nonionic ethoxylated and nonethoxylated surfactants, abietic acid, almond oil PEG, beeswax, butylglucoside caprate, C18-C36 acid glycol ester, C9-C15 alkyl phosphate, caprylic/capric triglyceride PEG-4 esters, ceteareth-7, cetyl alcohol, cetyl phosphate, corn oil PEG esters, DEA-cetyl phosphate, dextrin laurate, dilaureth-7 citrate, dimyristyl phosphate, glycereth-17 cocoate, glyceryl erucate, glyceryl laurate, hydrogenated castor oil PEG esters, isosteareth-11 carboxylic acid, lecithin, lysolecithin, nonoxynol-9, octyldodeceth-20, palm glyceride, PEG diisostearate, PEG stearamine, poloxamines, polyglyceryls, potassium linoleate, PPG's, raffinose myristate, sodium caproyl lactylate, sodium caprylate, sodium cocoate, sodium isostearate, sodium tocopheryl phosphate, steareths, TEA-C12-C13 pareth-3 sulfate, tri-C12-C15 pareth-6 phosphate, and trideceths.

In another embodiment, the topical intra-oral composition may optionally include one or more penetration enhancing agents to increase absorption across the area of application. Exemplary penetration enhancing agents include dimethyl sulfoxide (DMSO), urea and substituted urea compounds. Examples of other suitable penetration enhancers include 2-methyl propan-2-ol, propan-2-ol, ethyl-2-hydroxypropanoate, hexan-2,5-diol, polyoxyethylene(2) ethyl ether, di(2-hydroxypropyl) ether, pentan-2,4-diol, acetone, polyoxyethylene(2) methyl ether, 2-hydroxypropionic acid, 2-hydroxyoctanoic acid, propan-1-ol, 1,4-dioxane, tetrahydrofuran, butan-1,4-diol, propylene glycol dipelargonate, polyoxypropylene 15 stearyl ether, octyl alcohol, polyoxyethylene ester of oleyl alcohol, oleyl alcohol, lauryl alcohol, dioctyl adipate, dicapryl adipate, di-isopropyl adipate, di-isopropyl sebacate, dibutyl sebacate, diethyl sebacate, dimethyl sebacate, dioctyl sebacate, dibutyl suberate, dioctyl azelate, dibenzyl sebacate, dibutyl phthalate, dibutyl azelate, ethyl myristate, dimethyl azelate, butyl myristate, dibutyl succinate, didecyl phthalate, decyl oleate, ethyl caproate, ethyl salicylate, isopropyl palmitate, ethyl laurate, 2-ethyl-hexyl pelargonate, isopropyl isostearate, butyl laurate, benzyl benzoate, butyl benzoate, hexyl laurate, ethyl caprate, ethyl caprylate, butyl stearate, benzyl salicylate, 2-hydroxypropanoic acid, 2-hydroxyoctanoic acid, dimethyl sulphoxide, N,N-dimethyl acetamide, N,N-dimethyl formamide, 2-pyrrolidone, 1-methyl-2-pyrrolidone, 5-methyl-2-pyrrolidone, 1,5-dimethyl-2-pyrrolidone, 1-ethyl-2-pyrrolidone, phosphine oxides, sugar esters, tetrahydrofurfural alcohol, urea, diethyl-m-toluamide, 1-dodecylazacyloheptan-2-one, omega three fatty acids and fish oils, and combinations thereof.

In certain applications, it may be desirable to thicken the topical intra-oral composition. Suitable examples of thickening or viscosity increasing agents may include agents such as: acrylamides copolymer, agarose, amylopectin, bentonite, calcium alginate, calcium carboxymethyl cellulose, carbomer, carboxymethyl chitin, cellulose gum, dextrin, gelatin, hydrogenated tallow, hydroxytheylcellulose, hydroxypropylcellulose, hydroxpropyl starch, magnesium alginate, methylcellulose, microcrystalline cellulose, pectin, various PEG's, polyacrylic acid, polymethacrylic acid, polyvinyl alcohol, various PPG's, sodium acrylates copolymer, sodium carrageenan, xanthan gum, and yeast beta-glucan.

In another embodiment, the topical intra-oral composition may include one or more buffers. In the context of the present invention, all customary buffers are suitable, such as phosphate buffer, carbonate buffer, acetate buffer, formate buffer, citrate buffer, tris buffer, tris-(hydroxymethyl)-amino methane, glycylglycine buffer, glycine buffer, sodium phosphate buffer, sodium hydrogen phosphate buffer, sodium dihydrogen phosphate buffer, potassium phosphate buffer, potassium hydrogen phosphate buffer, potassium dihydrogen phosphate buffer or pyrophosphate buffer or mixtures thereof. Suitable examples also include sodium carbonate buffer, potassium carbonate buffer, sodium hydrogen carbonate buffer or potassium hydrogen carbonate buffer, and combinations thereof.

For topical intra-oral compositions formulated as a spray to be applied to the one or more teeth of the subject, the composition may generally include one or more propellants. Suitable propellants may include propane, butane, isobutane, dimethyl ether, carbon dioxide, nitrous oxide, and combinations thereof.

In yet another aspect, the topical intra-oral compositions may include one or more binders. Non-limiting examples of binders suitable for the formulations of various embodiments include starches, pregelatinized starches, gelatin, polyvinylpyrolidone, cellulose, methylcellulose, sodium carboxymethylcellulose, ethylcellulose, polyacrylamides, polyvinyloxoazolidone, polyvinylalcohols, C12-C18 fatty acid alcohols, polyethylene glycol, polyols, saccharides, oligosaccharides, polypeptides, oligopeptides, and combinations thereof. The polypeptide may be any arrangement of amino acids ranging from about 100 to about 300,000 Daltons.

In still another aspect, the topical intra-oral compositions may include one or more fillers. Suitable examples of fillers include, but are not limited to carbohydrates, inorganic compounds, and polyvinylpirrolydone. Other non-limiting examples of fillers include dibasic calcium sulfate, tribasic calcium sulfate, starch, calcium carbonate, magnesium carbonate, microcrystalline cellulose, dibasic calcium phosphate, tribasic calcium phosphate, magnesium carbonate, magnesium oxide, calcium silicate, talc, modified starches, lactose, sucrose, mannitol, and sorbitol.

In a further aspect, the topical intra-oral compositions may incorporate one or more lubricants. Non-limiting examples of lubricants include magnesium stearate, calcium stearate, zinc stearate, hydrogenated vegetable oils, sterotex, polyoxyethylene monostearate, talc, polyethylene glycol, sodium benzoate, sodium lauryl sulfate, magnesium lauryl sulfate, and light mineral oil.

In still another aspect, the topical intra-oral compositions may incorporate one or more diluents. Diluents suitable for use include but are not limited to pharmaceutically acceptable saccharides such as sucrose, dextrose, lactose, microcrystalline cellulose, fructose, xylitol, and sorbitol; polyhydric alcohols; starches; pre-manufactured direct compression diluents; and mixtures of any of the foregoing.

In an additional embodiment, the topical intra-oral compositions may incorporate one or more flavor-modifying agents. Suitable flavor-modifying agents include but are not limited to synthetic flavor oils and flavoring aromatics and/or natural oils, extracts from plants, leaves, flowers, fruits, and combinations thereof. Other non-limiting examples of flavor-modifying agents include cinnamon oils, oil of wintergreen, peppermint oils, clover oil, hay oil, anise oil, eucalyptus, vanilla, citrus oils such as lemon oil, orange oil, grape and grapefruit oil, fruit essences including apple, peach, pear, strawberry, raspberry, cherry, plum, pineapple, and apricot.

In a further embodiment, the topical intra-oral compositions may include one or more sweeteners for enhancing the palatability of the composition. Non-limiting examples of sweeteners may include glucose (corn syrup), dextrose, invert sugar, fructose, and mixtures thereof (when not used as a carrier); saccharin and its various salts such as the sodium salt; dipeptide sweeteners such as aspartame; dihydrochalcone compounds, glycyrrhizin; Stevia rebaudiana (Stevioside); chloro derivatives of sucrose such as sucralose; sugar alcohols such as sorbitol, mannitol, sylitol, hydrogenated starch hydrolysates and the synthetic sweetener 3,6-dihydro-6-methyl-1,2,3-oxathiazin-4-one-2,2-dioxide, particularly the potassium salt (acesulfame-K), and sodium and calcium salts thereof.

In another embodiment, the topical intra-oral compositions may incorporate one or more coloring agents. Suitable coloring agents include but are not limited to food, drug and cosmetic colors (FD&C), drug and cosmetic colors (D&C), or external drug and cosmetic colors (Ext. D&C). These colors or dyes, along with their corresponding lakes, and certain natural and derived colorants may be suitable for use in various embodiments.

In still another embodiment, the topical intra-oral compositions of the current invention may include one or more taste-masking agents. Suitable examples of taste-masking agents include, but are not limited to cellulose hydroxypropyl ethers (HPC) such as Klucel®, Nisswo HPC and PrimaFlo HP22; low-substituted hydroxypropyl ethers (L-HPC); cellulose hydroxypropyl methyl ethers (HPMC) such as Seppifilm-LC, Pharmacoat®, Metolose SR, Opadry YS, PrimaFlo, MP3295A, Benecel MP824, and Benecel MP843; methylcellulose polymers such as Methocel® and Metolose®; Ethylcelluloses (EC) and mixtures thereof such as E461, Ethocel®, Aqualon®-EC, Surelease; Polyvinyl alcohol (PVA) such as Opadry AMB; hydroxyethylcelluloses such as Natrosol®; carboxymethylcelluloses and salts of carboxymethylcelluloses (CMC) such as Aqualon®-CMC; polyvinyl alcohol and polyethylene glycol co-polymers such as Kollicoat IR®; monoglycerides (Myverol), triglycerides (KLX), polyethylene glycols, modified food starch, acrylic polymers and mixtures of acrylic polymers with cellulose ethers such as Eudragit® EPO, Eudragit® RD100, and Eudragit® E100; cellulose acetate phthalate; sepifilms such as mixtures of HPMC and stearic acid, cyclodextrins, and mixtures of these materials. In other embodiments, additional taste-masking agents contemplated are those described in U.S. Pat. Nos. 4,851,226, 5,075,114, and 5,876,759.

The skilled artisan will understand that the topical intra-oral compositions of the current disclosure may be administered in any dosage form capable of adequately contacting the composition with the area to be imaged. For instance, suitable examples of dosage forms that may incorporate the topical intra-oral composition may include, but are not limited to powders, solutions, suspensions, oils, creams, liquids, ointments, gels, lotions, sprays, pastes, foams, mouth rinses, dentifrices, and films. Regardless of the dosage form, chosen, the topical intra-oral composition should not be detrimental to the health of the subject and thus free of hazardous and toxic components being able to migrate out of the composition. Additionally, the dosage form incorporating the topical intra-oral composition must generally be capable of resisting degradation at ambient conditions. Ambient conditions may, for example, include pressures of about 900 to about 1100 mbar, temperatures of about -10 to about 60° C, and relative humidity ranging from about 10 to about 100%.

The topical intra-oral compositions and dosage forms of the current disclosure may also be incorporated into a pre-made kit for use by practitioners. The may include (1) a topical intra-oral composition as described herein, incorporated into any of the dosage forms described herein; and (2) an application accessory. The application accessory may be a container, a syringe, a sprayer, a brush, a swab, a tray, or combinations thereof. The application accessory may be any suitable size. The kit may include more than one application accessory or more than one kind of application accessory (i.e., a syringe and a brush). The kit may also comprise instructions for using the kit.

In addition, the kits of the current disclosure can be provided as a one-compartment system or a multi-compartment system. In a one-compartment system, all components of the composition are contained in a single dosage form packed in an appropriate application accessory for use by a practitioner. Alternatively, in a multi-compartment system the various components of the topical intra-oral composition may be stored in separate containers to be combined into a single application accessory prior to administration. In another aspect, the various components of the topical intra-oral composition stored in separate containers do not need to be combined in a single application accessory, and may instead be applied simultaneously via one or more separate containers.

### II. Method of Applying the Topical Intra-Oral Compositions

As noted, in one aspect, the current disclosure comprises a method of imaging dental caries in a subject comprising the steps of: (a) administering a topical intra-oral composition comprising a contrast agent and a pharmaceutically acceptable carrier to one or more teeth of the subject; and (b) producing a radiographic image of the one or more teeth. Generally, the topical intra-oral solutions of the current invention may be used on any subject in need of dental imaging. The subject may include any human or non-human animal.

Typically, the topical intra-oral composition is applied to a tooth surface of the subject. The method of administering the topical intra-oral composition to one or more teeth may include administration means such as dropping, immersing, inserting, rinsing, spraying, air-blasting, brushing, swabbing, or combinations thereof. In some embodiments, the teeth may be dried prior to administering the topical intra-oral composition. Methods of drying teeth are known in the art, and may include blasting air or a propellant onto the surface of the teeth until they are dry.

In one embodiment, the methods of imaging dental caries of the current invention generally comprise administering and applying the topical intra-oral composition for an amount of time sufficient to allow for the absorption of the composition into the dental caries. Generally, as noted in **FIGS. 1-3****,** minimal time periods are required for the topical intra-oral composition to accumulate in the oral or dental tissue. Typically, the amount of time required for the topical intra-oral composition to accumulate and provide enhanced radiographic images ranges from about 1 second to about 30 minutes. One of the beneficial properties of the current invention is the fast accumulation time, such that no significant delay between administration of the composition and imaging is required. After applying the topical intra-oral composition for the appropriate amount of time, the excess composition may generally be removed to avoid unnecessary noise or false suggestions of cavities. For instance, the teeth may be wiped to remove the excess composition.

In addition, the current invention may comprise administering the topical intra-oral composition in an amount sufficient to allow for adequate coverage and accumulation in the tissue to be imaged. The skilled artisan will understand that the amount and volume of the topical intra-oral composition applied to the oral surface will depend on the dosage form in which the composition has been incorporated. For instance, the amount of a topical intra-oral solution applied to the one or more teeth will vary from the amount of a topical intra-oral foam or paste applied to the one or more teeth.

The subsequent radiographic imaging step described herein refer to producing a radiographic image via radiographic exposure, and may include any radiographic imaging process known in the art. Suitable examples of radiographic imaging methods that may be used with the current invention include, but are not limited, to computed tomography, and intra- and extra-oral x-ray imaging, including dental panoramic tomography.

As used herein, "a", "an", "the", "at least one" and "one or more" are used interchangeably. The terms "comprises" or "contains" and variations thereof do not have a limiting meaning where these terms appear in the description and claims. Also herein, the recitations of numerical ranges by endpoints include all numbers subsumed within that range.

All matter contained in the above description and in the examples given below, shall be interpreted as illustrative.

### EXAMPLES

The following examples demonstrate various aspects of the invention.

### Example 1: Absorption of Topical Intra-Oral Composition Compared to Control

A radiographic contrast imaging analysis using x-ray imaging was performed, comparing the imaging of identified dental caries before and after administration of a topical intra-oral composition comprising sodium iodide and distilled water according to the methods of the current invention. Specifically, an x-ray image of a tooth with identified dental caries was performed, without administering the topical intra-oral composition. This image was used as a control for determining the ability of typical x-ray images to detect dental caries in a patient. Subsequently, the same tooth was administered a composition comprising sodium iodide and distilled water, with the composition having a sodium iodide concentration of 9 molar. After administration of the composition to the tooth, x-ray images were obtained 20 seconds after administration and 16 minutes after administration. Further, an x-ray image of the tooth was taken 14 days after administration of the composition to determine if the composition continued to reside in the dental carious lesion. The images obtained are found in FIG. 1.

As shown in FIG. 1, the image of the tooth after administration of the topical 9 M sodium iodide solution provided an enhanced radiographic image of dental caries after 20 seconds and 16 minutes, as evidenced by the white opaque sections of the images found on the left side of the tooth. The control image was able to detect the presence of a carious lesion but could not distinguish if it was cavitated or non-cavitated. Thus, the experimental results of FIG. 1 illustrate that the methods and compositions of the current invention provide enhanced imaging of dental caries in teeth, images that would not otherwise be captured by typical x-ray processes. Additionally, as noted in FIG. 1, after 14 days the topical intra-oral composition had an altered x-ray appearance. The central white enamel area seen at 20 seconds and 16 minutes post application now had a central darker region of the enamel which corresponded to the cavity space and a diffuse white region in the dentin. This change suggests that the composition is ultimately absorbed from the cavity space into the dentin revealing the true extent of the lesion which is not seen in the control x-ray image.

### Example 2: Comparison of Absorption and Imaging Between Cavitated Surfaces and Sound Healthy Surfaces

An experiment was performed to test the ability of the topical intraoral compositions and methods to differentiate between cavitated surfaces (dental caries) and healthy, sound surfaces. Specifically, an x-ray image of a tooth with an identified cavitated surface on one side of the tooth and a sound surface on the other side was performed, without administering the topical intra-oral composition. This image was used as a control for determining the ability of the topical intra-oral compositions to selectively absorb into cavitated surfaces, rather than sound surfaces. Subsequently, the same tooth was administered a composition comprising sodium iodide and distilled water, with the composition having a sodium iodide concentration of 9 molar. After administration of the composition to the tooth, an x-ray image was obtained 2 minutes after administration. The results are illustrated in FIG. 2.

As shown in FIG. 2, the control image without the administration of the topical intra-oral composition did detect the carious lesion but could not distinguish between it being a cavitated or non-cavitated lesion. The control correctly identified the sound surface on the left side of the tooth and there being a carious lesion on the right side of the tooth. However, the image obtained from the tooth administered the topical intra-oral composition was able to identify the carious lesion as a cavitated surface 2 minutes after administration, as evidenced by the white, opaque sections found on the side of the tooth. Importantly, the composition did not absorb into the healthy, sound surface found on the left side of the tooth, as evidenced by the lack of a white, opaque section. Therefore, the images in FIG. 2 illustrate selective absorption of the composition in cavitated surfaces, rather than sound, healthy surfaces, enabling the practitioner to distinguish between cavitated surfaces requiring therapeutic intervention and sound, healthy surfaces that do not require therapeutic intervention.

### Example 3: Comparison of Absorption and Imaging between Cavitated Lesions and Non-Cavitated Lesions

An experiment was performed to test the ability of the topical intra-oral compositions and methods to differentiate between cavitated surfaces (a cavity) requiring treatment and non-cavitated lesions not requiring treatment. Specifically, an x-ray image of a tooth with an identified cavitated surface on the right side of the tooth and a non-cavitated lesion on the other side was performed, without administering the topical intra-oral composition. This image was used as a control for determining the ability of the topical intra-oral compositions to selectively absorb into cavitated lesions, rather than non-cavitated lesions. Subsequently, the same tooth was administered a composition comprising sodium iodide and distilled water, with the composition having a sodium iodide concentration of 9 molar. After administration of the composition to the tooth, an x-ray image was obtained 3 minutes after administration. The results are illustrated in FIG. 3.

As shown in FIG. 3, the control image without the administration of the topical intra-oral composition did detect a large cavitated carious lesion on the right as a large dark radiolucency and a non-cavitated carious lesion on the left as a faint dark radiolucency. However, radiographically the cavitated and non-cavitated lesions had no unique characteristics which would allow the practitioner to distinguish between the non-cavitated lesion on the left side of the tooth and the cavitated lesion on the right side of the tooth. The image from the tooth 3 minutes after administration of the topical intra-oral composition shows the presence of the cavitated surface on the right side of the tooth, as evidenced by the white plaque seen in the image. However, the image does not provide enhanced radiographic contrast for the non-cavitated lesion on the left side of the tooth. Accordingly, the images in FIG. 3 illustrate selective absorption of the topical intra-oral composition in cavitated lesions, rather than non-cavitated lesions, enabling the practitioner to distinguish between cavitated surfaces requiring therapeutic intervention and non-cavitated lesions that may not require therapeutic intervention.

### Example 4. Non-Ionic Iodine Compounds as Caries Contrast Agents.

An experiment was performed to compare the ability of ionic and non-ionic topical intra-oral compositions comprising iodide to provide an enhanced radiographic image of dental caries. A 9M solution of sodium iodide (an ionic contrast agent) and a 76% solution of iopamidol (a non-ionic contrast agent) were administered. Each compound was applied in turn under identical conditions to the same tooth cavity and the tooth was radiographically analyzed as discussed above. After application and testing of each compound, the contrast was washed out from the tooth, and the tooth was tested radiographically to ensure there was no contrast remaining. Both ionic and non-ionic classes of iodine compounds produced radiographic contrast enhancement revealing tooth cavitation (FIG. 4).

### Example 5. Barium Compounds as Caries Contrast Agents. (reference)

An experiment was performed to test the ability of barium compounds to provide an enhanced radiographic image of dental caries. A barium sulfate solution comprising 1 gram of barium sulfate in 0.5 ml distilled water was administered. The compound was applied, and the tooth was analyzed and compared to other contrast agents and to the control treated tooth as discussed in Example 4. After application and testing of each compound, the contrast was washed out from the tooth and the tooth was tested radiographically to ensure there was no contrast remaining. The barium sulfate compound produced radiographic contrast enhancement revealing tooth cavitation (FIG. 4).

### Example 6. Barium Compounds as Caries Contrast Agents. (reference)

An experiment may be performed to test the ability of solutions comprising gold, iothalamate meglumine, calcium hydroxide and zinc oxide to provide an enhanced radiographic image of dental caries. The compound may be applied and the tooth may be analyzed and compared to other contrast agents and to the control treated tooth as discussed in Examples 4 and 5.

## Claims

1. A topical intra-oral solution comprising a contrasting agent for use in the *in vivo* diagnosis of cavitated dental caries; wherein the contrasting agent is an iodide compound; and wherein the topical intra-oral solution is administered to one or more teeth of a subject and a radiographic image of the one or more teeth is produced as part of the diagnosis.

2. The topical intra-oral solution for use according to claim 1, wherein the iodide compound is selected from the group consisting of sodium iodide, potassium iodide, magnesium iodide, calcium iodide, diatrizoate, and combinations thereof.

3. The topical intra-oral solution for use according to claim 1 or claim 2, wherein the iodide compound is selected from the group consisting of sodium iodide and potassium iodide.

4. The topical intra-oral solution for use according to any of claims 1 to 3, wherein the iodide compound is sodium iodide.

5. The topical intra-oral solution for use according to any of claims 1 to 4, wherein the topical intra-oral solution includes water.

6. The topical intra-oral solution for use according to claim 5, wherein the water includes at least one member of a group consisting of: deionized water, distilled water, and purified water.

7. The topical intra-oral solution for use according to claim 1, wherein the iodide compound is present in the topical intra-oral solution at a concentration ranging from about 0.5 molar to about 9 molar.

8. The topical intra-oral solution for use according to claim 1, wherein the iodide compound is present in the topical intra-oral solution at a concentration ranging from about 2 molar to about 8 molar.

9. The topical intra-oral solution for use according to claim 1, wherein the iodide compound is present in the topical intra-oral solution at a concentration ranging from about 4 molar to about 7 molar.

10. The topical intra-oral solution for use according to claim 1, wherein the iodide compound is present in the topical intra-oral solution at a concentration ranging from about 5.5 molar to about 6.5 molar.

11. The topical intra-oral solution for use according to claim 1, wherein the iodide compound is present in the topical intra-oral solution at a concentration of about 6 molar.

12. The topical intra-oral solution for use according to any of the above claims, wherein after applying the topical intra-oral solution to a tooth, allowing an about 1 second to about 30 minute opportunity for the topical intra-oral solution to absorb before producing the radio-graphic image of the tooth as part of the diagnosis.

13. The topical intra-oral solution for use according to any of the above claims, wherein the radio-graphic image includes a white opaque region produced from the contrasting agent when the tooth includes cavitated dental caries.

## Patentansprüche

1. Topische intraorale Lösung, umfassend ein Kontrastmittel zur Verwendung bei der *In-vivo*-Diagnose von kavitierter Zahnkaries; wobei das Kontrastmittel eine Iodidverbindung ist; und wobei die topische intraorale Lösung an einen Zahn oder mehr Zähne eines Patienten verabreicht wird und eine Röntgenaufnahme von dem einen Zahn oder mehr Zähnen als Teil der Diagnose angefertigt wird.

2. Topische intraorale Lösung zur Verwendung nach Anspruch 1, wobei die Iodidverbindung aus der Gruppe ausgewählt ist, bestehend aus Natriumiodid, Kaliumiodid, Magnesiumiodid, Calciumiodid, Diatrizoat und Kombinationen davon.

3. Topische intraorale Lösung zur Verwendung nach Anspruch 1 oder Anspruch 2, wobei die Iodidverbindung aus der Gruppe ausgewählt ist, bestehend aus Natriumiodid und Kaliumiodid.

4. Topische intraorale Lösung zur Verwendung nach einem der Ansprüche 1 bis 3, wobei die Iodidverbindung Natriumiodid ist.

5. Topische intraorale Lösung zur Verwendung nach einem der Ansprüche 1 bis 4, wobei die topische intraorale Lösung Wasser enthält.

6. Topische intraorale Lösung zur Verwendung nach Anspruch 5, wobei das Wasser mindestens ein Glied einer Gruppe enthält, bestehend aus: deionisiertem Wasser, destilliertem Wasser und gereinigtem Wasser.

7. Topische intraorale Lösung zur Verwendung nach Anspruch 1, wobei die Iodidverbindung in der topischen intraoralen Lösung in einer molaren Konzentration im Bereich von ca. 0,5 bis ca. 9 vorliegt.

8. Topische intraorale Lösung zur Verwendung nach Anspruch 1, wobei die Iodidverbindung in der topischen intraoralen Lösung in einer molaren Konzentration im Bereich von ca. 2 bis ca. 8 vorliegt.

9. Topische intraorale Lösung zur Verwendung nach Anspruch 1, wobei die Iodidverbindung in der topischen intraoralen Lösung in einer molaren Konzentration im Bereich von ca. 4 bis ca. 7 vorliegt.

10. Topische intraorale Lösung zur Verwendung nach Anspruch 1, wobei die Iodidverbindung in der topischen intraoralen Lösung in einer molaren Konzentration im Bereich von ca. 5,5 bis ca. 6,5 vorliegt.

11. Topische intraorale Lösung zur Verwendung nach Anspruch 1, wobei die Iodidverbindung in der topischen intraoralen Lösung in einer molaren Konzentration von ca. 6 vorliegt.

12. Topische intraorale Lösung zur Verwendung nach einem der vorstehenden Ansprüche, wobei nach Applikation der topischen intraoralen Lösung auf einen Zahn, der topischen intraoralen Lösung vor Anfertigung der Röntgenaufnahme von dem Zahn als Teil der Diagnose eine ca. 1-sekündige bis ca. 30-minütige Gelegenheit gegeben wird, absorbiert zu werden.

13. Topische intraorale Lösung zur Verwendung nach einem der vorstehenden Ansprüche, wobei die Röntgenaufnahme einen vom Kontrastmittel produzierten weißen opaken Bereich enthält, wenn der Zahn von kavitierter Zahnkaries befallen ist.

## Revendications

1. Solution intra-orale topique comprenant un agent de contraste pour une utilisation dans le diagnostic in vivo de carries dentaires avec cavités ; dans laquelle l'agent de contraste est un composé iodure ; et dans laquelle la solution intra-orale topique est administrée à une ou plusieurs dents d'un sujet et une image radiographique desdites une ou plusieurs dents est produite dans le cadre du diagnostic.

2. Solution intra-orale topique pour une utilisation selon la revendication 1, dans laquelle le composé iodure est choisi dans le groupe comprenant l'iodure de sodium, l'iodure de potassium, l'iodure de magnésium, l'iodure de calcium, le diatrizoate et les combinaisons de ceux-ci.

3. Solution intra-orale topique pour une utilisation selon la revendication 1 ou la revendication 2, dans laquelle le composé iodure est choisi dans le groupe comprenant l'iodure de sodium et l'iodure de potassium.

4. Solution intra-orale topique pour une utilisation selon l'une quelconque des revendications 1 à 3, dans laquelle le composé iodure est l'iodure de sodium.

5. Solution intra-orale topique pour une utilisation selon l'une quelconque des revendications 1 à 4, dans laquelle la solution intra-orale topique contient de l'eau.

6. Solution intra-orale topique pour une utilisation selon la revendication 5, dans laquelle l'eau contient au moins un élément d'un groupe comprenant : l'eau déminéralisée, l'eau distillée et l'eau purifiée.

7. Solution intra-orale topique pour une utilisation selon la revendication 1, dans laquelle le composé iodure est présent dans la solution intra-orale topique dans une concentration comprise dans l'intervalle d'environ 0,5 M à environ 9 M.

8. Solution intra-orale topique pour une utilisation selon la revendication 1, dans laquelle le composé iodure est présent dans la solution intra-orale topique dans une concentration comprise dans l'intervalle d'environ 2 M à environ 8 M.

9. Solution intra-orale topique pour une utilisation selon la revendication 1, dans laquelle le composé iodure est présent dans la solution intra-orale topique dans une concentration comprise dans l'intervalle d'environ 4 M à environ 7 M.

10. Solution intra-orale topique pour une utilisation selon la revendication 1, dans laquelle le composé iodure est présent dans la solution intra-orale topique dans une concentration comprise dans l'intervalle d'environ 5,5 M à environ 6,5 M.

11. Solution intra-orale topique pour une utilisation selon la revendication 1, dans laquelle le composé iodure est présent dans la solution intra-orale topique dans une concentration d'environ 6 M.

12. Solution intra-orale topique selon l'une quelconque des revendications précédentes, dans laquelle après l'application de la solution intra-orale topique sur une dent, une opportunité d'environ 1 seconde à environ 30 minutes est laissée pour que la solution intra-orale topique soit absorbée avant que l'image radiographique de la dent soit produite dans le cadre du diagnostic.

13. Solution intra-orale topique selon l'une quelconque des revendications précédentes, dans laquelle l'image radiographique contient une région opaque blanche produite grâce à l'agent de contraste quand la dent contient des carries dentaires avec cavités.
